# EUROPÄISCHE PATENTSCHRIFT

(11) **EP 1 326 817 B1**
(45) Veröffentlichungstag und Bekanntmachung des Hinweises auf die Patenterteilung: **15.11.2006**
(21) Anmeldenummer: 01974322.8
(22) Anmeldetag: 02.10.2001
(51) Int. Cl.: C07C 15/24

(54) **VERFAHREN UND VORRICHTUNGEN ZUR GEWINNUNG VON NAPHTHALIN AUS KOKSOFENROHGAS**
METHOD AND DEVICES FOR PRODUCING NAPHTHALENE FROM COKE PRODUCED FROM COKE OVEN CRUDE GAS
PROCEDE ET DISPOSITIFS POUR PRODUIRE DE LA NAPHTALINE A PARTIR DE GAZ BRUT DE COKERIE

(30) Priorität: 17.10.2000 DE 10051349
(43) Veröffentlichungstag der Anmeldung: 16.07.2003
(73) Patentinhaber: Deutsche Montan Technologie GmbH, 45307 Essen (DE)
(72) Erfinder: ROSSA, Frank, 44803 Bochum (DE); GIERTZ, Hans-Josef, 40880 Ratingen (DE); SCHRÖDER, Horst, 44388 Dortmund (DE)
(86) Internationale Anmeldenummer: PCT/EP2001/011361
(87) Internationale Veröffentlichungsnummer: WO 2002/032840

(56) Entgegenhaltungen:
- DE-A- 4 012 146
- GB-A- 894 203
- DATABASE WPI Section Ch, Week 197441 Derwent Publications Ltd., London, GB; Class H09, AN 1974-72036V XP002184781 & JP 49 034721 B (NIPPON STEEL CORP), 17. September 1974 (1974-09-17)
- DATABASE WPI Section Ch, Week 197732 Derwent Publications Ltd., London, GB; Class E14, AN 1977-56563Y XP002184782 & JP 52 077043 A (KAWASAKI JUKOGYO KK), 29. Juni 1977 (1977-06-29)

## Beschreibung

Die Erfindung betrifft ein Verfahren zur Gewinnung von Naphthalin aus Koksofenrohgas gemäß dem Oberbegriff des Anspruchs 1 sowie dafür geeignete Vorrichtungen.

Bei der thermischen Behandlung von Kohle unter Luftabschluss in einem Koksofen entsteht ein Koksofenrohgas, das mittels Berieselungswasser in einer Rohgasvorlage auf ca. 80 °C abgekühlt und mit Wasserdampf gesättigt wird. Dabei werden etwa 20 % des in dem Koksofenrohgas mitgeführten Naphthalins in der flüssigen Phase an Teer gebunden. Die restlichen 80 % des Naphthalins werden anschließend in einer indirekten Rohgaskühlung gemeinsam mit teerigen und wässrigen Bestandteilen in Vorkühlern kondensiert. Diese Kondensate werden zunächst zur Rohgasvorlage gefördert, dort auf ca. 80 °C erwärmt und einer Teerscheidung zugeführt, in der die Trennung in Rohteer und Kohlewasser erfolgt. Das in dem Rohteer enthaltene Naphthalin wird aus dem Rohteer in verfahrenstechnisch sehr aufwendigen Prozessen gewonnen. Zunächst wird in einer Teerdestillation ein Naphthalinöl hergestellt, das anschließend einer Kristallisation zugeführt wird, in der Rohnaphthalin abgeschieden wird. Zur Gewinnung von Reinnaphthalin sind noch weitere Verfahren wie chemische Reaktionen (z.B. mit Natronlauge und Schwefelsäure) erforderlich oder das Rohnaphthalin wird erneut destilliert.

Die DE 40 12 146 A1 offenbart ein Verfahren zur indirekten und/oder direkten Vorkühlung von Koksofenrohgas. Dabei wird das Koksofenrohgas durch einen Elektrofilter geleitet. Anschließend wird es durch zwei Kühlstufen geleitet, zwischen denen ein Naphthalinwäscher angeordnet ist. Dieser Naphthalinwäscher wird als konventioneller Naphthalinwäscher mit Benzolwaschöl betrieben. Das mit Naphthalin angereicherte Benzolwaschöl des Naphthalinwäschers wird einer Benzolwaschöl-Regenerierung zugeführt, in der ein mit BTX-Aromaten angereichertes naphthalinhaltiges Produkt anfällt, das weiteren Verarbeitungsschritten zugeführt wird. Eine Gewinnung von Naphthalin ist nicht vorgesehen.

Der Erfindung liegt die Aufgabe zugrunde, ein Verfahren zur Gewinnung von Naphthalin aus Koksofenrohgas sowie dafür geeignete Vorrichtungen bereitzustellen, bei dem das Naphthalin in hoher Reinheit mit geringem Aufwand auf einfache Art und Weise gewonnen werden kann.

Diese Aufgabe wird durch die Merkmale des Anspruchs 1 gelöst.

Weiterbildungen erfolgen gemäß den Merkmalen der Unteransprüche.

Gemäß der Erfindung wird das aus den Koksöfen kommende Rohgas, wie üblich, mit Hilfe von Berieselungswasser in der Rohgasvorlage direkt auf ca. 80 °C gekühlt. Dabei entsteht eine flüssige und eine gasförmige Phase. Die flüssige Phase wird zur Teerscheidung geleitet, wo die Trennung in Teer und Kohlewasser erfolgt. Ein Teil des Kohlewassers wird wieder zur direkten Rohgaskühlung als Berieselungswasser eingesetzt. Die gasförmige Phase wird durch einen Elektrofilter geleitet, in dem der Feststoff- und Teergehalt des Koksofenrohgases bei einer Temperatur von ca. 80 °C um 99,99 % reduziert wird.

Da die Abscheidung in dem Elektrofilter bei einer Temperatur von ca. 80 °C stattfindet, verbleibt das (gasförmig vorliegende) Naphthalin beim Passieren des Elektrofilters in dem Koksofenrohgas. Das Koksofenrohgas wird nun von ca. 80 °C auf ca. 20 °C gekühlt, dabei wird das in dem Rohgas enthaltene Naphthalin abgeschieden. Da die Feststoffe sowie der Teer bereits in dem Elektrofilter abgeschieden worden sind, handelt es sich bei dem Naphthalin um ein sehr reines Naphthalin, das als verkaufsfähiges Produkt direkt einem Verbraucher zugeführt werden kann.

Damit das Naphthalin in dem Elektrofilter nicht abgeschieden wird, wird das Elektrofilter isoliert oder sogar beheizt, so dass in dem Elektrofilter isotherme Bedingungen von ca. 80 °C vorliegen. Da bei dem erfindungsgemäßen Verfahren der abgeschiedene Teer die inneren Oberflächen des Elektrofilters vor Korrosion schützt, kann der Elektrofilter aus einfachem Baustahl (z. B. ST 37-3) bestehen.

Der in dem Elektrofilter abgeschiedene Teer ist besonders naphthalinarm, da, wie oben dargestellt, das Naphthalin in dem Koksofenrohgas verbleibt. Dieser napthalinarme Teer wird zur Teerscheidung gefördert. Aufgrund des geringen Naphthalingehaltes (ca. 2 % Restnaphthalingehalt) wird die Emulsionsbildung in der Teer-Wasser-Scheidung verringert und so die Teerscheidung verbessert. Außerdem wird die benötigte Verweilzeit zur Teer-Wasser-Scheidung reduziert. Die Teer-Wasser-Scheidung kann auch bei etwas niedrigerer Temperatur durchgeführt werden, da die Emulsionsbildung verringert wird.

Gemäß einer Verfahrensvariante ist es auch möglich, den naphthalinarmen Teer aus dem Elektrofilter, da er nahezu wasserfrei ist, nicht zur Teerscheidung zu leiten, sondern direkt zum Teerlagerbehälter zu führen. Dadurch wird die Teerscheidung entlastet. Der naphthalinarme Teer kann auch in einem gesonderten Teerlagerbehälter gelagert werden und für andere Verfahren, wie z.B. die Vorkühlerspülung, verwendet werden.

Das Naphthalin kann aus dem Rohgas hinter dem Elektrofilter durch direkte oder indirekte Kühlung gewonnen werden.

Bei der direkten Kühlung wird das Koksofenrohgas nach dem Elektrofilter mit Wasser direkt gekühlt. Dabei scheidet sich das Naphthalin ab und wird anschließend von dem Wasser getrennt. Da das Wasser nahezu keine Verunreinigungen enthält, kann es im Kreislauf mit entsprechender Rückkühlung gefahren werden. Die Rückkühlung des Kreislaufwassers kann direkt oder indirekt erfolgen. Das Überschusswasser wird in die Vorlage geleitet.

Bei der indirekten Kühlung kristallisiert das Naphthalin an den Kühlflächen aus.

Das auskristallisierte Naphthalin kann auf verschiedene Art und Weise gewonnen werden:

Eine Möglichkeit ist, das Naphthalin durch Aufwärmen zu gewinnen. Dabei kann die Aufwärmung direkt oder indirekt erfolgen. Die Kühlfläche kann durch ein heißes Medium sowohl auf der "Naphthalinseite" als auch auf der anderen Seite erwärmt werden und so das Naphthalin abgezogen und über beheizte Leitungen zur Verladung transportiert werden.

Die Aufwärmung kann auch durch Heizstäbe erfolgen, die an geeigneter Stelle in der Vorrichtung eingebaut werden.

Eine andere Möglichkeit ist, das Naphthalin mit bereits erwärmtem flüssigen Naphthalin "abzuspülen". Flüssiges Naphthalin kann auch zur indirekten Aufheizung eingesetzt werden.

Es ist auch möglich, das auskristallisierte Naphthalin mit einem Lösungsmittel von der Kühlfläche abzulösen und anschließend zu gewinnen.

Die Gewinnung des auskristallisierten Naphthalins kann auch mechanisch, z. B. mit Hilfe von Schabern erfolgen. Dabei kann entweder der Schaber oder die Kühlfläche zur Gewinnung des Naphthalins bewegt werden.

Es ist auch möglich, das auskristallisierte Naphthalin mit Hilfe von mechanischen Schwingungen, wie sie durch einen Klopfer oder Rüttler oder mit Hilfe eines Unwuchtmotors erzeugt werden können, zu gewinnen.

Bei den zuvor beschriebenen Verfahren erfolgt die Gewinnung von Naphthalin diskontinuierlich, d.h. in einem ersten Verfahrensschritt wird das Naphthalin auskristallisiert und in einem zweiten Verfahrensschritt wird das auskristallisierte Naphthalin gewonnen.

Es ist jedoch auch möglich, die Gewinnung von Naphthalin kontinuierlich bzw. quasikontinuierlich durchzuführen. Bei dieser Fahrweise wird eine Vorrichtung segmentweise im Kühlbetrieb bzw. im Naphthalingewinnungsbetrieb gefahren. Ein kontinuierlicher Betrieb ist auch bei Einsatz von Schabern oder Schwingungserzeugern möglich.

Für die Durchführung des erfindungsgemäßen Verfahrens muss lediglich ein Elektrofilter und eine für die Abscheidung von Naphthalin geeignete Vorrichtung vorgesehen werden, aus der dann das abgeschiedene Naphthalin gewonnen werden kann.

Für eine Nachrüstung auf einer bestehenden Kokerei ist es möglich, lediglich zusätzliche Elektrofilter vorzusehen und die vorhandenen Vorkühler zu nutzen. Sind Elektrofilter nach den Vorkühlern vorhanden, so könnten diese Elektrofilter in den Rohgasweg vor die Vorkühler eingebunden werden. Elektrofilter nach der Vorkühlung erübrigen sich bei den erfindungsgemäßen Verfahren.

Da bei bestehenden Kokereien in der Regel mehrere Vorkühler vorhanden sind, kann das Verfahren wie folgt mit den vorhandenen Vorkühlern durchgeführt werden:

Eine Gruppe von z. B. zwei Vorkühlern wird zum Auskristallisieren des Naphthalins verwendet. Die restlichen Vorkühler laufen im üblichen Vorkühlerbetrieb mit Teerspülung. Bei einem quasikontinuierlichen Betrieb wird z. B. ein Vorkühler zum Auskristallisieren und ein Vorkühler zur Naphthalingewinnung verwendet. Bei einem Anstieg des Druckverlustes im Vorkühler zur Naphthalingewinnung oder bei einer Erhöhung der Rohgasaustrittstemperatur um maximal 2 °C wird das Rohgas nicht mehr durch diesen Vorkühler geleitet, sondern durch den nach der Naphthalingewinnung im Stand-by-Betrieb stehenden Vorkühler.

Das auf den Kühlrohren auskristallisierte Naphthalin wird durch Aufwärmen gewonnen. Dabei können die Kühlrohre von innen oder außen aufgewärmt werden. Eine Kombination von Innen- und Außenaufwärmung ist ebenfalls möglich. Nachdem das Naphthalin aus den Vorkühlern gewonnen wurde, erfolgt eine Umstellung und die Vorkühler werden wieder zum Auskristallisieren des Naphthalins verwendet und das Naphthalin wird aus den anderen Vorkühlern gewonnen.

Bei dieser Verfahrensweise kann ohne Probleme auf eine geringere Naphthalingewinnung umgestellt werden und teilweise oder auch vollständig auf den klassischen Vorkühlerbetrieb umgestellt werden. Bei diesem Vorkühlerbetrieb empfiehlt es sich, damit Naphthalin nicht die Vorkühler verstopft, die Vorkühler mit dem naphthalinarmen Teer aus dem Elektrofilter zu spülen.

Bei dem erfindungsgemäßen Verfahren zur Gewinnung von Naphthalin aus Koksofenrohgas kann somit als geeignete Vorrichtung zur Gewinnung des Naphthalins ein konventioneller Vorkühler verwendet werden. In dem Kühlschritt kristallisiert das Naphthalin an den Kühlrohren aus der Gasphase aus, anschließend kann das Naphthalin durch die oben beschriebenen Verfahren gewonnen werden. Während der Naphthalingewinnung wird das Koksofenrohgas durch einen im Stand-by-Betrieb stehenden weiteren Vorkühler geleitet.

Der Vorkühler kann auch für eine quasikontinuierliche Naphthalingewinnung umgerüstet werden. Dazu muss der Vorkühler segmentweise mit ansteuerbaren Ventilen ausgestattet werden. Auf diese Art und Weise ist es möglich, in dem Vorkühler gleichzeitig einen Kühlbetrieb für das Auskristallisieren des Naphthalins und einen Naphthalingewinnungsbetrieb mit Aufwärmen des Naphthalins durchzuführen.

Zur Gewinnung des Naphthalins sind auch andere Vorrichtungen möglich. Es muss lediglich eine Kühlfläche vorhanden sein, von der mit den oben beschriebenen Verfahren das Naphthalin gewonnen werden kann. Als Kühlfläche kann z. B. eine Trommel oder eine Scheibe verwendet werden. Wird diese Trommel oder diese Scheibe mit mindestens einem Schaber ausgestattet, so kann die Naphthalingewinnung auch kontinuierlich erfolgen.

Es ist auch möglich, das Naphthalin in einem Rohrkühler, dessen Rohre an der Außenwand gekühlt werden, auszukristallisieren. Das Naphthalin scheidet sich dann an der Rohrinnenwand ab und kann mit den oben beschriebenen Verfahren gewonnen werden.

Die vorgenannten sowie die beanspruchten und in den Ausführungsbeispielen beschriebenen erfindungsgemäß zu verwendenden Bauteile unterliegen in ihrer Größe, Formgestaltung, Materialauswahl und technischen Konzeption keinen besonderen Ausnahmebedingungen, so dass die in dem Anwendungsgebiet bekannten Auswahlkriterien uneingeschränkt Anwendung finden können.

Weitere Einzelheiten, Merkmale und Vorteile des Gegenstandes der Erfindung ergeben sich aus den Unteransprüchen sowie aus der nachfolgenden Beschreibung der zugehörigen Zeichnung, in der beispielhaft bevorzugte Ausführungsformen des erfindungsgemäßen Verfahrens dargestellt sind.

In der Zeichnung zeigen:
- Figur 1: eine schematische Darstellung des Verfahrens zur Gewinnung von Naphthalin aus Koksofenrohgas mit direkter Kühlung,
- Figur 2: eine schematische Darstellung des Verfahrens zur Gewinnung von Naphthalin aus Koksofenrohgas mit indirekter Kühlung und
- Figur 3: eine schematische Darstellung der Verfahrensvariante gemäß der neben der Gewinnung von Naphthalin ebenfalls die konventionelle Rohgaskühlung durchgeführt werden kann.

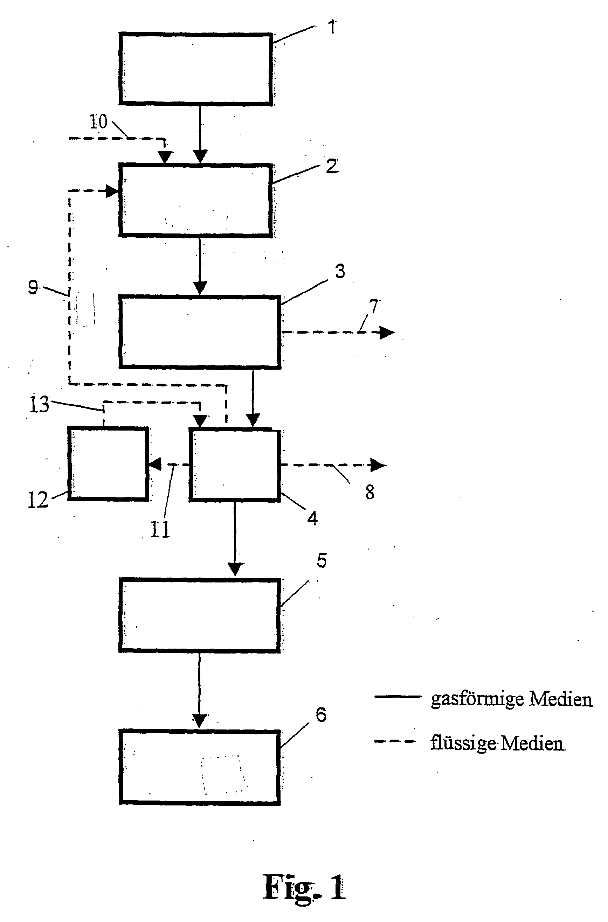

Aus der Figur 1 geht hervor, dass ein aus Koksöfen 1 kommendes Rohgas in einer Rohgasvorlage 2 mit über eine Leitung 10 zugeführten Berieselungswasser von ca. 800 °C auf ca. 80 °C gekühlt wird. Das Koksofenrohgas wird in einen Elektrofilter 3 geleitet, in dem der Feststoff- und Teergehalt des Koksofenrohgases um 99,99 % reduziert wird. Der abgeschiedene Teer wird über eine Leitung 7 zu der Teerscheidung oder zu dem Teersammelbehälter gefördert. Das Koksofenrohgas wird in eine Vorrichtung 4 zum Abscheiden des Naphthalins geleitet, in der das Naphthalin durch direkte Kühlung mit Wasser abgeschieden und vom Wasser getrennt wird. Das Naphthalin wird über eine Leitung 8 zu einer nicht dargestellten Trennvorrichtung geleitet, in der das Naphthalin von den an ihm haftenden Restwasser getrennt wird. Dieses Restwasser kann dem Kühlwasser zugeführt werden. Das Kühlwasser wird über eine Leitung 11, einen Kühler 12 und einer Leitung 13 im Kreislauf gefahren. Überschusswasser wird über eine Leitung 9 zu der Rohgasvorlage 2 geleitet. Das Koksofenrohgas wird zu einer Gasreinigung 5 gefördert, wo es von Schwefelwasserstoff, Ammoniak und gegebenenfalls von Benzol befreit wird, bevor es zu einem Koksofengasverbraucher 6 gelangt.

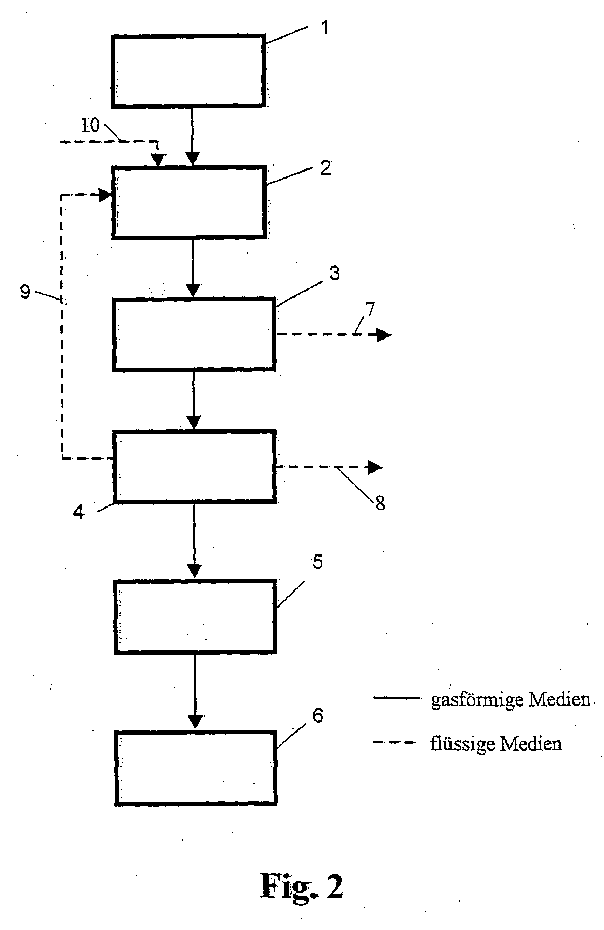

In der Figur 2 ist die Verfahrensvariante mit indirekter Kühlung zur Gewinnung des Naphthalins dargestellt. Die Bezugszeichen haben die gleiche Bedeutung wie in der Figur 1. Das Koksofenrohgas wird aus dem Elektrofilter 3 in eine Vorrichtung 4 zum Auskristallisieren des Naphthalins geleitet, in der das Naphthalin an den Kühlflächen abgeschieden wird, während der auskondensierte Wasserdampf über die Leitung 9 zu der Rohgasvorlage 2 geleitet wird. Das auf den Kühlflächen auskristallisierte Naphthalin wird durch Erwärmung der Kühlflächen als flüssiges Naphthalin aus der Vorrichtung 4 abgezogen und über eine beheizte Leitung 8 zu einer nicht dargestellten Verladung transportiert.

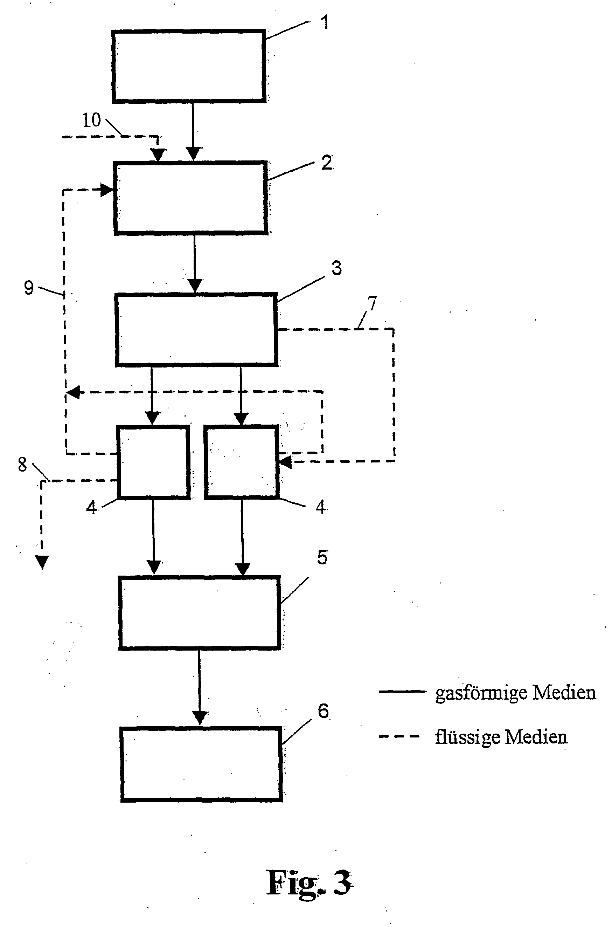

In der Figur 3 ist ein Verfahrensschema dargestellt, in dem zwei Vorkühler 4 und 4' als zum Auskristallisieren des Naphthalins verwendet werden können.. Die Vorrichtung 4 wird zur Naphthalingewinnung verwendet, d.h. über die Leitung 8 wird das Reinnaphthalin zur Verladung transportiert. Die Vorrichtung 4' wird im Vorkühlerbetrieb gefahren. Dabei wird der naphthalinarme Teer über Leitung 7 zur Spülung des Vorkühlers 4' verwendet, um Verstopfungen durch Naphthalin zu verhindern. Die übrigen Bezugszeichen haben die gleiche Bedeutung wie in der Figur 1.

### Bezugszeichenliste

- 1: Koksöfen
- 2: Vorlage
- 3: Elektrofilter
- 4: Vorrichtung zum Abscheiden
- 4': Vorrichtung zum Kühlen
- 5: Koksofengasreinigung
- 6: Koksofengasverbraucher
- 7: Leitung (Teer)
- 8: Leitung (Naphthalin)
- 9: Leitung (Kondensate)
- 10: Leitung (Berieselungswasser)
- 11: Leitung (Kühlwasser)
- 12: Kühler
- 13: Leitung (Kühlwasserrückführung)

## Patentansprüche

1. Verfahren zur Gewinnung von Naphthalin aus Koksofenrohgas, bei dem das Koksofenrohgas mittels Berieselungswasser direkt gekühlt und durch anschließende Kühlung und Aufarbeitung das Naphthalin gewonnen wird, **dadurch gekennzeichnet, dass** das Koksofenrohgas nach der direkten Koksofenrohgaskühlung durch einen Elektrofilter geleitet wird und anschließend derart gekühlt wird, dass das in dem Koksofenrohgas enthaltene Naphthalin aus der Gasphase abgeschieden und ohne weitere Aufarbeitung als Rein-Naphthalin gewonnen wird.

2. Verfahren nach Anspruch 1, **dadurch gekennzeichnet, dass** das Elektrofilter derart isoliert oder beheizt wird, dass es isotherm arbeitet.

3. Verfahren nach den Ansprüchen 1 und 2, **dadurch gekennzeichnet, dass** die Kühlung des Koksofenrohgases nach dem Elektrofilter direkt erfolgt.

4. Verfahren nach den Ansprüchen 1 und 2, **dadurch gekennzeichnet, dass** die Kühlung des Koksofenrohgases nach dem Elektrofilter indirekt erfolgt.

5. Verfahren nach den Ansprüchen 1 bis 4, **dadurch gekennzeichnet, dass** das abgeschiedene Naphthalin durch Aufwärmen gewonnen wird.

6. Verfahren nach Anspruch 5, **dadurch gekennzeichnet, dass** die Aufwärmung indirekt erfolgt.

7. Verfahren nach Anspruch 5, **dadurch gekennzeichnet, dass** die Aufwärmung direkt erfolgt.

8. Verfahren nach Anspruch 6 und 7, **dadurch gekennzeichnet, dass** die Aufwärmung mit bereits erwärmtem flüssigen Naphthalin durchgeführt wird.

9. Verfahren nach Anspruch 1 und 2, **dadurch gekennzeichnet, dass** das abgeschiedene Naphthalin mit einem Lösungsmittel gelöst und anschließend das Lösungsmittel entfernt wird.

10. Verfahren nach Anspruch 1 und 2, **dadurch gekennzeichnet, dass** das abgeschiedene Naphthalin mechanisch gewonnen wird.

11. Verfahren nach Anspruch 1 und 2, **dadurch gekennzeichnet, dass** das abgeschiedene Naphthalin kontinuierlich gewonnen wird.

12. Vorrichtung zur Durchführung des Verfahrens nach Anspruch 1, **dadurch gekennzeichnet, dass** ein konventioneller Vorkühler, in dem Heizstäbe vorgesehen sind, als Vorrichtung zum Abscheiden des Naphthalins verwendet wird.

13. Vorrichtung zur Durchführung des Verfahrens nach Anspruch 1, **dadurch gekennzeichnet, dass** ein Vorkühler segmentweise mit ansteuerbaren Ventilen ausgestattet ist.

14. Vorrichtung zur Durchführundes Verfahrens nach Anspruch 1, **dadurch gekennzeichnet, dass** eine Kühlfläche mit mindestens einem Schaber vorgesehen ist.

15. Vorrichtung zur Durchführung des Verfahrens nach Anspruch 1, **dadurch gekennzeichnet, dass** eine gekühlte Trommel mit Schabern ausgestattet ist.

16. Vorrichtung zur Durchführung des Verfahrens nach Anspruch 1, **dadurch gekennzeichnet, dass** eine gekühlte Scheibe mit Schabern ausgestattet ist.

17. Vorrichtung zur Durchführung des Verfahrens nach Anspruch 1, **dadurch gekennzeichnet, dass** an einer Kühlfläche mindestens ein Schwingungserzeuger vorgesehen ist.

## Claims

1. Method for recovering naphthalene from coke oven crude gas, in which the coke oven crude gas is directly cooled by means of trickling water and through subsequent cooling and processing the naphthalene is recovered, **characterised in that** the coke oven crude gas, after the direct coke oven crude gas cooling, is passed through an electrostatic filter and then so cooled that the naphthalene contained in the coke oven crude gas is separated out from the gas phase and without further processing is recovered as pure naphthalene.

2. Method according to Claim 1, **characterised in that** the electrostatic filter is so insulated or heated that it operates isothermally.

3. Method according to Claims 1 and 2, **characterised in that** the cooling of the coke oven crude gas following the electrostatic filter is effected directly.

4. Method according to Claims 1 and 2, **characterised in that** the cooling of the coke oven crude gas following the electrostatic filter is effected indirectly.

5. Method according to Claims 1 to 4, **characterised in that** the separated-out naphthalene is recovered by heating up.

6. Method according to Claim 5, **characterised in that** the heating up is effected indirectly.

7. Method according to Claim 5, **characterised in that** the heating up is effected directly.

8. Method according to Claim 6 and 7, **characterised in that** the heating up is carried out with already heated liquid naphthalene.

9. Method according to Claim 1 and 2, **characterised in that** the separated-out naphthalene is dissolved with a solvent and the solvent is then removed.

10. Method according to Claim 1 and 2, **characterised in that** the separated-out naphthalene is mechanically recovered.

11. Method according to Claim 1 and 2, **characterised in that** the separated-out naphthalene is continuously recovered.

12. Apparatus for carrying out the method according to Claim 1, **characterised in that** a conventional precooler in which heating rods are provided is used for separating out the naphthalene.

13. Apparatus for carrying out the method according to Claim 1, **characterised in that** a precooler is equipped segmentwise with controllable valves.

14. Apparatus for carrying out the method according to Claim 1, **characterised in that** a cooling surface is provided with at least one scraper.

15. Apparatus for carrying out the method according to Claim 1, **characterised in that** a cooled drum is equipped with scrapers.

16. Apparatus for carrying out the method according to Claim 1, **characterised in that** a cooled disc is equipped with scrapers.

17. Apparatus for carrying out method according to Claim 1, **characterised in that** at least one vibration generator is provided at a cooling surface.

## Revendications

1. Procédé pour la récupération de naphtalène à partir du gaz de pyrogénation d'un four à coke, dans lequel le gaz de pyrogénation du four à coke est refroidi directement avec de l'eau de pulvérisation et est récupéré par refroidissement ultérieur et traitement du naphtalène, **caractérisé en ce que** le gaz de pyrogénation du four à coke est guidé, après le refroidissement direct du gaz de pyrogénation du four à coke, à travers un dépoussiéreur électrostatique et est ensuite refroidi de telle sorte que le naphtalène contenu dans le gaz de pyrogénation du four à coke est séparé de la phase gazeuse et est récupéré sous la forme de naphtalène pur en l'absence d'un traitement ultérieur.

2. Procédé selon la revendication 1, **caractérisé en ce que** le dépoussiéreur électrostatique est isolé ou chauffé de telle sorte qu'il travaille dans des conditions isothermiques.

3. Procédé selon les revendications 1 et 2, **caractérisé en ce que** le refroidissement du gaz de pyrogénation du four à coke a lieu de manière directe après le dépoussiéreur électrostatique.

4. Procédé selon les revendications 1 et 2, **caractérisé en ce que** le refroidissement du gaz de pyrogénation du four à coke a lieu de manière indirecte après le dépoussiéreur électrostatique.

5. Procédé selon les revendications 1 à 4, **caractérisé en ce que** le naphtalène séparé est récupéré par réchauffement.

6. Procédé selon la revendication 5, **caractérisé en ce que** le réchauffement a lieu de manière indirecte.

7. Procédé selon la revendication 5, **caractérisé en ce que** le réchauffement a lieu de manière directe.

8. Procédé selon les revendications 6 et 7, **caractérisé en ce que** le réchauffement est mis en oeuvre avec du naphtalène liquide déjà réchauffé.

9. Procédé selon les revendications 1 et 2, **caractérisé en ce que** le naphtalène séparé est dissous avec un solvant, le solvant étant éliminé par la suite.

10. Procédé selon les revendications 1 et 2, **caractérisé en ce que** le naphtalène séparé est récupéré par voie mécanique.

11. Procédé selon les revendications 1 et 2, **caractérisé en ce que** le naphtalène séparé est récupéré en continu.

12. Dispositif pour la mise en oeuvre du procédé selon la revendication 1, **caractérisé en ce qu'**on utilise un condenseur primaire conventionnel dans lequel on prévoit des barres de chauffage, à titre de dispositif pour la séparation du naphtalène.

13. Dispositif pour la mise en oeuvre du procédé selon la revendication 1, **caractérisé en ce qu'**un condenseur primaire est équipé, de manière segmentée, de soupapes réglables.

14. Dispositif pour la mise en oeuvre du procédé selon la revendication 1, **caractérisé en ce qu'**on prévoit une surface de refroidissement comprenant au moins un grattoir.

15. Dispositif pour la mise en oeuvre du procédé selon la revendication 1, **caractérisé en ce qu'**un tambour refroidi est équipé de grattoirs.

16. Dispositif pour la mise en oeuvre du procédé selon la revendication 1, **caractérisé en ce qu'**un disque refroidi est équipé de grattoirs.

17. Dispositif pour la mise en oeuvre du procédé selon la revendication 1, **caractérisé en ce qu'**on prévoit, sur la surface de refroidissement, au moins un générateur d'oscillations.
